(19) Europäisches Patentamt
European Patent Office
Office européen des brevets

(11) Veröffentlichungsnummer: **0 512 211 B1**

(12) **EUROPÄISCHE PATENTSCHRIFT**

(45) Veröffentlichungstag der Patentschrift: **12.04.95**

(51) Int. Cl.[6]: **C07C 231/02**, C07C 233/15, C07C 211/52

(21) Anmeldenummer: **92103519.2**

(22) Anmeldetag: **29.02.92**

# (54) Verfahren zur Herstellung von substituierten Malonesteraniliden und Malonsäure-monoaniliden.

(30) Priorität: **06.05.91 DE 4114733**

(43) Veröffentlichungstag der Anmeldung:
**11.11.92 Patentblatt 92/46**

(45) Bekanntmachung des Hinweises auf die Patenterteilung:
**12.04.95 Patentblatt 95/15**

(84) Benannte Vertragsstaaten:
**DE FR GB**

(56) Entgegenhaltungen:
**EP-A- 0 394 440**
**WO-A-87/05898**

**CHEMICAL ABSTRACTS, vol. 109, no. 9, 28. August 1988, Columbus, Ohio, US;abstract no. 68759d, N. SHINDO ET AL. 'Plant growth regulating properties of alkyl 2-(phenylcarbomoyl)propionates', seite 257**

**TETRAHEDRON, (INCL. TETRAHEDRON REPORTS) Bd. 44, Nr. 8, 1988, OXFORD GB Seiten 2351 - 2358 F. MARAN ET AL. 'Electro-Carboxylation of 2-Bromoisobutyramides. A Useful Synthetic Way to Ester-Amides of 2,2-Dimethylmalonic Acid'**

**PATENT ABSTRACTS OF JAPAN vol. 10, no. 292 (C-376)(2348) 3. Oktober 1986**

**CHEMICAL ABSTRACTS, vol. 87, no. 7, 15. August 1977, Columbus, Ohio, US;abstract no. 52961e, T. MUKAI ET AL. 'Phenylcarbamic acid ester' Seite 438 ;**

(73) Patentinhaber: **HÜLS AKTIENGESELLSCHAFT**

**D-45764 Marl (DE)**

(72) Erfinder: **Steffen, Klaus-Dieter, Dr.**
**Röckelstrasse 36**
**W-5202 Hennef 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Die Erfindung betrifft die Herstellung von wahlweise Monoester-monoaniliden, Monosäure-monoaniliden oder Bisaniliden der ein- oder zweifach substituierten Malonsäuren der allgemeinen Formel (I)

(I) 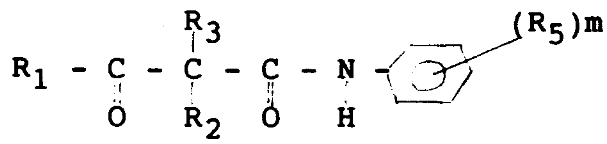

durch Umsetzung von ein- und zweifach substituierten Malonsäuredialkylestern der Formel (II) mit Aminen der Formel (III)

(II) 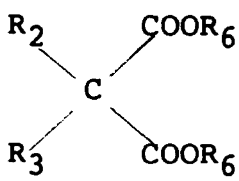

(III) 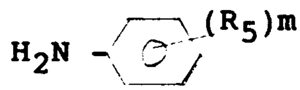

durch Zugabe von Alkalialkoholaten.

Die zum Teil bekannten (Houben-Weyl 11/2, S. 182), jedoch wenig beachteten Alkalisalze der Aniline (III) bilden sich erfindungsgemäß leicht aus dem Anilin und Alkalialkoholat.
Die Herstellung von Malon-monoester-monoanilid und Malonsäuremonoanilid erfolgt nach dem Schema:

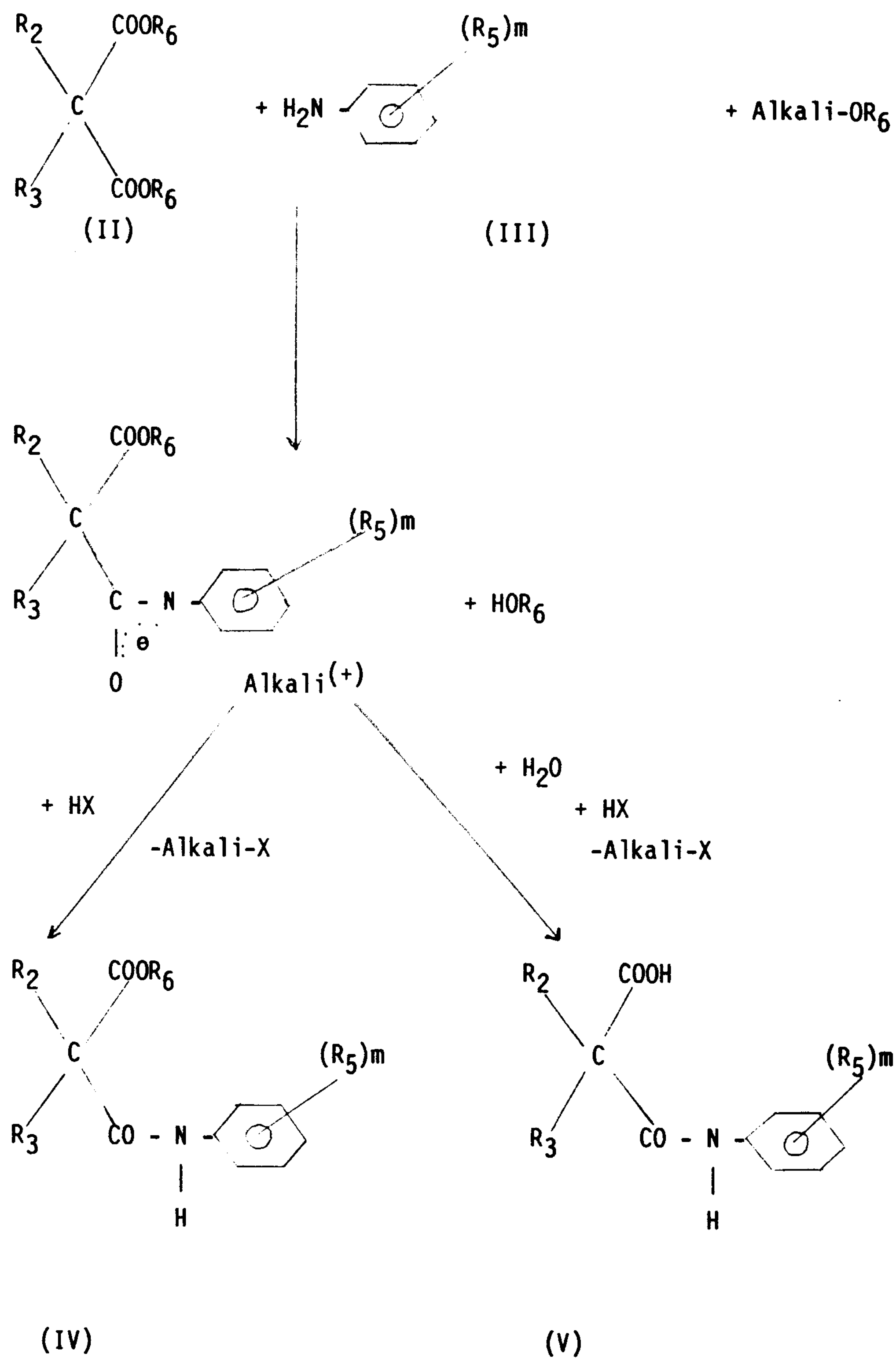


(II) (III) (IV) (V)

X = Säureanion

In den Formeln (I) bis (V) bedeuten

$R_1$ = O-Alkyl ($C_1$-$C_6$), OH, $O^-$ Alkali $^+$,

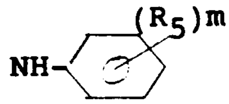

$R_2$ = Alkyl ($C_1$-$C_6$), Alkoxy ($C_1$-$C_6$), Aryl
$R_3$ = H, gleiche oder verschiedene $R_2$
$R_2$ + $R_3$ = $-(R_4CR_4)_n-$, n = 2-5
$R_4$ = H, $CH_3$, Halogen
$R_5$ = Halogen, Alkyl ($C_1$-$C_6$), Alkoxy ($C_1$-$C_6$), $N(R_6)_2$, Cyan, Acyl, Nitro, Thioalkyl ($C_1$-$C_6$)
m = 0 - 5
$R_6$ = Alkyl ($C_1$-$C_6$)

Verbindungen der Formel (I) sind zum Teil bekannt und werden als Pflanzenwuchs-Regulatoren (WO 87/05781) zur Erhöhung der Ernteerträge z.B. von Bohnen (WO 87/05897) oder zur Verzögerung des Pflanzenwuchses von Gras oder Bäumen (WO 87/05898) eingesetzt. Dort erfolgt die Herstellung aus unsubstituierten Malonestern ($R_2$ = $R_3$ = H) durch einseitige Verseifung, Bildung des Säurechlorids, Umsetzung mit dem Amin und Einfügung der Substituenten $R_2$ bzw. $R_3$. Dieser Weg über mehrere Stufen ist aufwendig und ergibt geringe Ausbeuten. Nach Beispiel 22 der WO 87/05898 wird Diethylmalonat direkt mit 4-Chloranilin umgesetzt, jedoch kann Malonsäure-monoethylester-mono-(4-chloranilid) nur in 20 %iger Ausbeute erhalten werden.

In ebenfalls geringen Ausbeuten von 28,7 % wird Malonsäure-bis(3,4-dichloranilid) durch Kochen von Diethylmalonat mit 3,4-Dichloranilin über 18 Std. erhalten (D.J. Beaver u.a. J.A.C.S. 79 (1957) 1236).

Die übliche Umsetzung von Estern mit Aminen zu den entsprechenden Säureamiden mit $NH_4Cl$ als Katalysator bringt keine Verbesserung. Auch katalytische Mengen von Alkalialkoholat haben keine oder negative Wirkung auf den Reaktionsverlauf und die Reinheit der Zielprodukte.

Nach F.D. Chattaway u.a. (J. chem. Soc. 97, S.339) wird der unsubstituierte Malonsäurediethylester mit verschiedenen Halogenanilinen in der Siedehitze (Kp. v. DEM: 199 °C) zu den Mono- und Bis-halogenaniliden umgesetzt. Unter diesen Bedingungen werden Bisanilide und Mono-anilide der Malonsäure nebeneinander erhalten. Obwohl Diethylmalonat bereits im Überschuß von 50 % zum Halogenanilin eingesetzt wird, sind Reinheit und Ausbeute unbefriedigend; die einzige angegebene Ausbeute beträgt 20 %.

Nach dem Stand der Technik werden aus Anilinen und Malonestern demnach Anilide der substituierten bzw. unsubstituierten Malonester und -säure nur in geringen Ausbeuten und geringer Reinheit erhalten. Es bestand daher die Aufgabe, die Spezifität der Reaktion zu erhöhen und Ausbeute sowie Reinheit der Produkte deutlich zu verbessern.

Gemäß der Erfindung wird der Malonester (II) ebenfalls direkt mit dem Anilin (III) zum jeweiligen Malon-monoester-monoanilid umgesetzt. Überraschend werden hohe Ausbeuten zum Teil weit über 90 % und hohe Reinheiten des primär gebildeten Monoester-monoanilids durch Zugabe von Alkalialkoholat erzielt.

Vorzugsweise wird zuerst aus dem jeweiligen Anilin (III) und einem Alkalialkoholat bzw. einem Erdalkalialkoholat das Alkalisalz des Anilins (III) gebildet und danach die Umsetzung mit dem Malonsäuredialkylester (II) vorgenommen.

Die Alkalisalze der Aniline (III) ermöglichen es, die Reaktion bei deutlich niedrigeren Temperaturen gegenüber dem Stand der Technik, nämlich bei 0 bis 150 °C, vorzugsweise bei 10 bis 50 °C und Nachreaktion bei 50 bis 150 °C auszuführen. Weiterhin bilden sich so die Alkalisalze der Malonsäure-monoester-monoanilide (IV) vollständig und rein bei einfacher Reaktionsführung. Die Menge des Alkalialkoholats soll im wesentlichen stöchiometrisch zu dem Anilin (III) sein, wobei ein Überschuß Alkoholat nicht schadet, aber nutzlos ist. Es können 85 bis 130, vorzugsweise 95 bis 110, sehr bevorzugt für praktische Zwecke 99 bis 105 Mol-% Alkalialkoholat, bezogen auf das Anilin (III), verwendet werden. Versuche entsprechend Beispiel 1 haben gezeigt, daß eine unterhalb 80 Mol-% verminderte Menge Alkoholat die Ausbeute von (IV) immer weiter senkt und eine steigende starke Verunreinigung von (IV) verursacht. Der Malonester wird in 95 bis 105 % der stöchiometrischen Menge des Anilins eingesetzt, vorzugsweise in genau stöchiometrischer Menge. Die Alkalisalze der Aniline (III) besonders der Chloraniline, entstehen einfach durch Erwärmen des Anilins mit alkoholischer Alkalialkoholat-Lösung bzw. -Suspension. Beim Erkalten scheiden sich große Kristalle der Alkali-anilide ab und können so gewonnen werden. Die Reinheit dieser Kristalle beträgt fast 100 % (Perchlorsäure-Titration, Na-Bestimmung, G.C.).

Die Alkalialkoholate werden daher bevorzugt in einem nicht wässrigen Lösungsmittel, sehr bevorzugt in Lösung des jeweilig zugehörigen Alkohols eingesetzt. Als Alkoholate kommen Natrium- oder Kaliumalkoholate derdaliphatischen Alkohole mit 1 bis 6 Kohlenstoffatomen, z.B. $NaOCH_3$, $KOCH_3$, $NaOC_2H_5$, K-t-Butylat

oder $Mg(OR_6)_2$ in Betracht. Das Alkalisalz des Anilins (III) kann in fester Form oder als Lösung mit dem ein- oder zweifach substituierten Malonester (II) in beliebiger Reihenfolge der Zugabe eingesetzt werden. Wurde zuvor das Alkalisalz des Anilins in Lösung hergestellt, erfolgt die Zugabe des Malonesters in dasselbe Gefäß.

Weitere Lösungsmittel z.B. aliphatische Kohlenwasserstoffe wie Heptan, Hexan, Cyclohexan, aromatische Kohlenwasserstoffe wie Toluol, Xylol oder Ether wie t-Butyl-methylether können bei der Reaktion anwesend sein.

Die Reaktion setzt bei Raumtemperatur ein und verläuft mit geringer Wärmetönung. Temperaturen über 30 °C lassen Nebenprodukte entstehen und sollen vermieden werden. Die Reaktion wird durch kurzes Erwärmen vervollständigt, wobei der Alkohol aus der Anilid-Kondensation mit weiteren zugesetzten Lösungsmitteln durch Destillation, oft als Azeotrop mit Methanol, gegebenenfalls unter vermindertem Druck, ganz oder weitgehend entfernt und das Lösemittel-Alkohol-Gemisch nach Auftrennung wiederverwendet wird.

Das Alkalisalz des jeweiligen Malon-monoester-monoanilids (IV) liegt jetzt als Schmelze, Suspension oder Lösung in einem Lösungsmittel vor, aus der es durch Abkühlen ausgeschieden und filtriert werden kann, was einer Reinigung durch Entfernen von restlichen Ausgangsstoffen oder Verunreinigungen gleichkommt.

Dem Alkalisalz von (IV) oder dessen Suspension in Lösungsmitteln werden zur Neutralisierung Säuren wie HCl, $H_2SO_4$, Essig- oder Ameisensäure in mindestens äquivalenter Menge zum vorhandenen Alkali zugesetzt. Die Alkalisalze der zugegebenen Säure werden in Wasser gelöst und aus dieser wässrigen Salz-Lösung wird die freie Verbindung (IV) durch Filtration oder Phasenabtrennung abgeschieden, gewaschen und als Produkt gewonnen. Es ist auch möglich, nach der Neutralisierung die lösemittelhaltige Reaktionsmischung von dem ausgefallenen Alkalisalz (z.B. NaCl) abzufiltrieren und das im Lösemittel gelöste Malonester-anilid nach Abdestillation des Lösemittels durch Kristallisation oder Vakuumdestillation zu gewinnen.

Nach dem Verfahren können zunächst Monoester-monoanilide und daraus ohne Zwischenisolierung der Vorstufe Monosäure-monoanilide der ein- oder mehrfach, d.h. durch $R^2$ und $R^3$ substituierten Malonsäuren in gleicher Weise hergestellt werden. Eine bestimmte Auswahl der Stoffe (I) wird jedoch als Agrochemikalien nach den genannten WO-Anmeldungen verwendet und bevorzugt nach der Erfindung hergestellt, nämlich einerseits Mono- und Bisanilide mit Chlorsubstituenten und andererseits beliebige Anilide von solchen Malonsäuren bzw. -estern, in welchen die Gruppen $R^2$ und $R^3$ gemeinsam die an das mittlere C-Atom der Malonsäure gebundene Gruppe $-(R_4CR_4)-$ mit n = 2 bis 5 bilden, besonders einen Cyclopropan- bzw. Cyclobutanring, der Methylgruppen oder Chlor enthalten kann. Weiter bevorzugt sind Anilide der Mono- bzw. Dialkyl-malonsäure bzw. des Monesters mit Alkyl = $C_1$ bis $C_2$. Sehr bevorzugt sind die Cyclopropyl-1,1-dicarbonsäure-monoanilide bzw. -dianilide bzw. Monoestermonoanilide mit $R_5$ = Chlor und m = 1 bis 3. Für die Herstellung des Malonsäure-amids (V) wird das Alkalisalz von (IV) oder vorzugsweise direkt das alkalische Reaktionsgemisch mit Wasser versetzt und die Estergruppe zur Säuregruppe verseift. Dies kann durch Rühren bei Raumtemperatur oder durch Kochen unter Rückflußbedingungen über mehrere Stunden erfolgen. Zur besseren Verseifung ist unter Umständen auch die Zugabe von Katalysatoren wie Phasentransfer-Katalysatoren notwendig. Die genauen Reaktionsbedingungen bis zur vollständigen Verseifung sind anhand des Verseifungsfortschritts mittels analytischer Kontrollen festzulegen.

Ist die Verseifung beendet, d.h. der pH-Wert der wäßrigen Lösung von ca. 14 auf ca. 10 abgefallen, wird die organische Phase, die aus dem Lösungsmittel z.B. Toluol oder Heptan und färbenden Verunreinigungen besteht, abgetrennt und die wäßrige Phase mit Säuren wie HCl auf pH = 2 angesäuert. Die wäßrige, alkalische Phase kann zur Abtrennung unlöslicher Beiprodukte vor der Ansäuerung einer Klärfiltration unterzogen werden. Für einen weiteren Reinigungsschritt kann die alkalisch-wäßrige Phase zusätzlich mit frischem, nicht mit Wasser mischbarem Lösungsmittel z.B. Toluol, Heptan, Cyclohexan extrahiert werden. Diese organischen Lösemittelphasen werden für Folgeansätze wiederverwendet, wobei man die Wasserspuren durch kurzfristige Andestillation azeotrop entfernt oder auch Trockenmittel wie Molekularsiebe verwendet.

Das abgeschiedene Zielprodukt (V), das meist kristallin ausfällt, wird abfiltriert, gewaschen und getrocknet und braucht nicht mehr nachgereinigt zu werden.

Ist das Zielprodukt (V) eine Flüssigkeit, so erfolgt eine Abtrennung und anschließend eine Reindestillation im Vakuum. Die so über zwei Stufen, in Ausbeuten von 85 bis 90 % d.Th., bezogen auf den Malonester (II), erhaltenen Produkte weisen Reinheiten von 95 bis 100 % auf.

Bisanilide der Formel (I) entstehen zum Teil - je nach Reaktionsführung - in geringen Mengen neben den Monoaniliden und können nach der alkalischen Verseifung der Monoestermonoanilide d.h. vor Zugabe von Säuren aus der wässrigen Phase abgetrennt werden. Durch Umsetzung von zwei Äquivalenten Anilin-Alkalisalz werden nur Bisamide erhalten.

Auch Alkalisalze, d.h. Monoalkalisalze von Monoesteraniliden und nach Verseifung Monoalkalisalze, wenn gewollt auch Dialkalisalze von Monosäure-monoaniliden können durch Entfernung der organischen Phase und Eindampfen bzw. nach Zugabe von weiterem Alkali erhalten werden. Mono- bzw. Dialkalisalze von Bisaniliden werden nach Entfernen der organischen Phase durch Teilneutralisierung bzw. ohne Neutralisierung erhalten. Als Alkalisalze sind Na-Salze stark bevorzugt.

Beispiel 1

**Cyclopropyl-1,1-dicarbonsäure-mono(2,4-dichlor)-anilid**

**[Cyclopropyl-1-(2,4-dichlorphenylamino)carbonyl-1-carbonsäure]**

In einen 1,5 l-Vierhalskolben, versehen mit Thermometer, Rührer, kurzer Kolonne und Destillationsteil, werden 79,3 g 30 Gew.-%ige Natrium-methanolat-Lösung (0,44 Mol), 64,8 g 2,4-Dichloranilin (DCA, 0,4 Mol) und 300 ml Toluol eingefüllt und zum Sieden erhitzt. Das Methanol/Toluol-Azeotrop wird solange abdestilliert, bis die Kopftemperatur auf 110 °C angestiegen ist (90 g Destillat).
Nach dem Abkühlen auf 20 °C werden 63,2 g Cyclopropyl-1,1-dicarbonsäuredimethylester (CDM, 0,4 Mol) unter Rühren zudosiert, wobei die Temperatur auf 35 °C ansteigt. Es wird 1 Std. bei Raumtemperatur gerührt, dann wird Methanol mit Toluol azeotrop entfernt und noch 1,5 Std. zum Sieden erhitzt. Anschließend wird angekühlt, dann werden 500 ml Wasser zugegeben und die Ester-Gruppe durch Erhitzen bis zum Siedepunkt in 3,5 Std. verseift.
Nach dem Abkühlen wird die organische Toluolphase von der wäßrigen Phase, die einen pH-Wert von 10 bis 11 aufweist, abgetrennt. Die wäßrige Phase wird filtriert, dann mit konz. HCl auf pH = 3 angesäuert. Der weiße Niederschlag des Zielprodukts wird abfiltriert, mit Wasser gewaschen und bei 100 °C im Vakuum getrocknet.
Auswaage: 97,8 g (89,2 % d.Th. bzg. a. CDM)
Schmelzpunkt: 192 bis 193,5 °C
Reinheit (HPLC): 98,6 %
Die Toluolphase (150 g), die 0,8 bis 0,9 % 2,4-Dichloranilin, Reste an CDM und nicht verseiften Cyclopropyldicarbonsäuremonomethylester-monodichloranilid enthält, wird über Molekularsieb-Perlen getrocknet und für die folgenden Ansätze wieder eingesetzt.

Beispiele 2 und 3

**Cyclopropyl-1,1-dicarbonsäure-mono(2,4-dichlor)-anilid**

Wie in Beispiel 1 beschrieben, werden in derselben Apparatur 79,3 g 30 Gew.-%ige Natrium-methanolat-Lösung (0,44 Mol) mit nur 63,7 g 2,4-Dichloranilin und 124 g der aus Beispiel 1 abgetrennten Toluolphase, in der noch 1,1 g DCA gelöst sind, umgesetzt. An frischem Toluol werden noch 155 ml (Restmenge zu 300 ml) zugesetzt.
Nach Abdestillation des Methanols werden bei Raumtemperatur 63,2 g CDM (0,4 Mol) zugegeben und die Reaktion, wie in Beispiel 1 beschrieben, zum Zielprodukt zu Ende geführt.
Auswaage: 96,8 g (88,3 % d.Th. bzg. a. CDM)
Schmelzpunkt: 192 bis 193 °C
Reinheit (HPLC): 98,6 %
Im Beispiel 3 wird die Toluolphase, die im Beispiel 2 abgetrennt worden ist und noch 1,5 g Dichloranilin enthält, wieder eingesetzt neben 63,3 g frischem 2,4-Dichloranilin und weiteren 200 ml frischem Toluol.
Die Reaktionsführung ist dieselbe wie in Beispiel 1 und 2 beschrieben.
Auswaage: 96,8 g (88,3 % d.Th. bzg. a. CDM)
Schmelzpunkt: 191 bis 193 °C
Reinheit (HPLC): 98,4 %

Beispiel 4

**Cyclopropyl-1,1-dicarbonsäure-monomethylester-mono(2,4-dichlor)-anilid**

**[Cyclopropyl-1-(2,4-dichlorphenylamino)carbonyl-1-carbonsäuremethylester] und dessen Na-Salz.**

a) In einem 1,5 l-Vierhalskolben, versehen mit Thermometer, Rührer, kurzer Kolonne und Destillationsteil werden 79,3 g 30 Gew.-%ige $NaOCH_3$-Lösung (0,44 Mol), 64,8 g 2,4-Dichloranilin (0,4 Mol) und 500 ml Cyclohexan eingefüllt und zum Sieden erhitzt. Das Methanol wird als Azeotrop bei einer Siedetemperatur von 55 °C vollständig abdestilliert. Nach dem Abkühlen auf Raumtemperatur sind 63,2 g Cyclopropyl-1,1-dicarbonsäuredimethylester (CDM, 0,4 Mol) mit 200 ml Cyclohexan zuzugeben und anschließend ist aufzuheizen, bis alles Reaktionsmethanol abdestilliert ist. Nach 1,5 h wird abgekühlt und der ausgefallene Niederschlag [Na-Salz von Cyclopropyl-1,1-dicarbonsäure-monomethylester-mono(2,4-dichlor)-anilid] abfiltriert, mit Cyclohexan gewaschen und getrocknet.
Auswaage: 119,4 g (96,3 % d. Th.)

| $C_{12}H_{10}Cl_2NO_3Na$ (310,11) | | | | |
|---|---|---|---|---|
| ber.<br>gef. | C 46,5<br>C 46,4 | H 3,3<br>H 3,4 | N 4,5<br>N 4,5 | 0 15,5<br>0 15,3 |

Schmelzpunkt: > 220 °C (Zers.)
b) 115 g des unter a) hergestellten Na-Salzes werden in 1 l kaltem Wasser unter Rühren schnell gelöst (pH = 13 bis 14) und mit 20 g Ameisensäure auf pH = 4 bis 5 angesäuert. Der weiße Niederschlag wird abfiltriert, mit Wasser gewaschen und getrocknet.
Auswaage: 102,0 g (95,4 % d. Th.)
Eine aus Äthanol umkristallisierte Probe weist einen Schmelzpunkt von 136 bis 137,5 °C auf.
Reinheit: 98,1 % (HPLC)

Beispiel 5

**Cyclopropyl-1,1-dicarbonsäure-monoethylester-mono-(2,4-dichlor)-anilid**

**[Cyclopropyl-1-(2,4-dichlorphenylamino)carbonyl-1-carbonsäureethylester] und dessen Na-Salz sowie K-Salz**

In einem 1,5 l-Vierhalskolben, versehen mit Thermometer, Rührer, kurzer Kolonne und Destillationsteil, werden 59,9 g festes Natriumethanolat (0,88 Mol), 500 ml Cyclohexan und 129,6 g 2,4-Dichloranilin (0,8 Mol) zum Sieden erhitzt, wobei das Ethanol-Azeotrop abdestilliert. Nach dem Abkühlen auf Raumtemperatur werden 148,9 g Cyclopropyl-1,1-dicarbonsäurediethylester (CDE, 0,8 Mol) zugegeben und wieder zum Sieden erhitzt unter Abdestillation des gesamten Ethanols.
Nach 2 Std. wird abgekühlt und der Niederschlag (Na-Salz von Cyclopropyl-1,1-dicarbonsäure-monoethylester-mono-(2,4-dichlor)-anilid abfiltriert und getrocknet.
Das gesamte Produkt wird in 1 l kaltem Wasser aufgerührt und mit Ameisensäure auf einen pH-Wert von 4 bis 5 angesäuert. Der erhaltene Niederschlag wird filtriert, mit Wasser gewaschen und getrocknet.
Auswaage: 201 g (83,2 % d.Th.)
Schmelzpunkt: 95 bis 97 °C
Reinheit (HPLC): 97,1 %
In entsprechender Weise, jedoch mit einer 40 Gew.%igen Suspension von $KOC_2H_5$ in Ethanol wird das obengenannte K-Salz hergestellt.
Fp über 200 °C (unter Zersetzung)
Durch Lösen in Wasser und Ansäuern mit Ameisensäure wird das freie Monoester-monoanilid mit Reinheit und Schmelzpunkt wie oben erhalten.

Beispiel 6

**Cyclopropyl-1,1-dicarbonsäure-bis-(2,4-dichlor)-anilid**

**[N,N'-Bis-(2,4-dichlorphenyl)-cycloproyl-1,1-dicarbonsäureamid]**

In einem Vierhalskolben, versehen mit Thermometer, Rührer und Destillationsteil, werden 150 g 30 Gew.-%ige $NaOCH_3$-Lösung (0,83 Mol), 133 g 2,4-Dichloranilin (0,82 Mol) und 500 ml Toluol vorgelegt und in der Siedehitze das Methanol/Toluol-Azeotrop abdestilliert. Nach Ankühlung auf Raumtemperatur werden 63,2 g CDM (0,4 Mol) zudosiert und nach ca. 1 Std. nochmals für 1,5 Std. zum Sieden erhitzt, wobei das Methanol-Azeotrop wieder abgenommen wird. Nach dem Abkühlen wird mit Ameisensäure neutralisiert, der Niederschlag abfiltriert, mit Toluol und Wasser gewaschen sowie getrocknet.
Ausbeute: 202 g (90 % d. Th.)
Schmelzpunkt: 158 °C
Reinheit (HPLC): 99 %

| $C_{17}H_{12}Cl_4N_2O_2$ (418,11) | | | | |
|---|---|---|---|---|
| ber.<br>gef. | C 48,8<br>C 49,1 | H 2,9<br>H 3,1 | N 6,7<br>N 6,3 | 0 7,7<br>0 8,0 |

Beispiel 7

**Cyclopropyl-1,1-dicarbonsäure-monoanilid**

**Cyclopropyl-1,1-dicarbonsäure-bisanilid und deren Na-Salze**

a) Herstellung mit $NaOCH_3$
In einem Vierhalskolben, versehen mit Thermometer, Rührer, Kolonne und Destillationsteil, werden 79,2 g 30 Gew.-%ige $NaOCH_3$-Lösung (0,44 Mol) und 37,3 g Anilin (0,4 Mol) mit 500 ml Cyclohexan zum Sieden erhitzt und das Methanol als Azeotrop restlos abdestilliert. Nach dem Abkühlen auf Raumtemperatur werden 63,2 g Cyclopropyl-1,1-dicarbonsäure-dimethylester (0,4 Mol) zügig zusdosiert und nach 30 Min. wieder zum Sieden erhitzt unter Abdestillation des Methanol-Azeotrops. Nach 2 Std. Reaktionszeit wird abgekühlt und mit 500 ml Wasser 4 Std. zum Sieden erhitzt, bis der pH-Wert von ursprünglich 13 bis 14 auf 10 bis 11 abgefallen ist. Nach dem Abkühlen wird die organische Phase von der wäßrigen getrennt, die wäßrige Phase klarfiltiert und mit Ameisensäure auf einen pH-Wert von 3 bis 4 angesäuert. Der ausgefallene Niederschlag wird filtriert, gewaschen und getrocknet: Cyclopropyl-1,1-dicarbonsäure-mono-anilid.
Auswaage: 70,0 g (85,3 % d.Th.)
Schmelzpunkt: 181 bis 182 °C (umkrist.a. Cyclohexan)
Reinheit (HPLC): 98,6 %
b) Herstellung mit Kalium-t-butylat
Wie unter a) beschrieben, wird die gleiche Reaktion mit 0,4 Mol Anilin durchgeführt mit den Unterschieden, daß anstelle der $NaOCH_3$-Lösung äquivalente Mengen festes Kalium-t-butylat und statt Cyclohexan die gleiche Menge Toluol eingesetzt wird.
Nach der Verseifung mit Wasser und Trennung der Toluol-Phase von der $H_2O$-Phase wird die wäßrige Phase vom angefallenen Niederschlag abfiltriert; dieser wird mit Wasser neutral gewaschen und getrocknet: Cyclopropyl-1,1-dicarbonsäurebisanilid.
Auswaage: 13,4 g (11,9 % d.Th.)
Schmelzpunkt: 212 bis 213,5 °C
Reinheit: 97 % (HPLC)
Nach dem Ansäuern der filtrierten wäßrigen Phase mit Salzsäure auf pH = 3 fällt Cyclopropyl-1,1-dicarbonsäure-monoanilid mit über 80 %iger Ausbeute aus, dessen physikalische Daten bereits unter a) beschrieben sind.

Das nach a) erhaltene Monosäure-monoanilid und das nach b) erhaltene Bisanilid werden jeweils in Wasser durch Erwärmen unter Zusatz von einem Äquivalent bzw. zwei Äquivalenten NaOH gelöst. Beim Erkalten und Zusatz von Methanol scheiden sich die Mono-Na-Salze bzw. die Di-Na-Salze des Bisanilids

ab. Fp. über 200 ° C unter Zersetzung. Die Struktur wird durch Bildung der freien Verbindung durch Fällung mit Säure aus wässriger Lösung und Prüfung der Schmelzpunkte bewiesen.

Beispiele 8 bis 15

Unter den gleichen Bedingungen, nach denen Cyclopropyl-1,1-dicarbonsäure-monomethylester-mono-(2,4-dichlor)anilid (Beispiel 4) und Cyclopropyl-1,1-dicarbonsäure-mono-(2,4-dichlor)anilid (Beispiel 1) hergestellt worden sind, wurde Cyclopropyl-1,1-dicarbonsäuredimethylester (CDM) mit 3,4-Dichloranilin (3,4-DCA), 2,6-Dichloranilin (2,6-DCA), 2,5-Dichloranilin (2,5-DCA) und 3-Chloranilin (3-CA) zu den entsprechenden Cyclopropyl-ester- und -säureaniliden umgesetzt.
In nachfolgender Tabelle sind die Schmelzpunkte und G.C.-Reinheiten dieser Verbindungen aufgeführt.

Beispiele 8 - 15

| Ester / Säure \ x = | 3,4-Dichloranilid | | | 2,6-Dichloranilid | | | 2,3-Dichloranilid | | | 3-Chloranilid | | |
|---|---|---|---|---|---|---|---|---|---|---|---|---|
| | Beisp. | Fp [°C] | G.C.-Reinh. [%] | Beisp. | Fp [°C] | G.C.-Reinh. [%] | Beisp. | Fp [°C] | G.C.-Reinh. [%] | Beisp. | Fp [°C] | G.C.-Reinh. [%] |
| $COOCH_3$ / CO-x | 8 | 157-59 | 93 1) | 10 | 100-02 | 96,0 | 12 | 113-14 | 98,0 | 14 | 82-85 | 96,1 |
| COOH / CO-x | 9 | 219-21 | 98,1 | 11 | 237-39 | 97,0 | 13 | 220-22 | 97,0 | 15 | 168-70 | 98,6 |
| Umkristallisation aus | Toluol | | | Toluol | | | Toluol | | | Cyclohexan | | |

1) mit entspr. Säure-Anteilen

Beispiel 16

**Diethylmalonsäure-monoethylester-(2,4-dichlor)-anilid**

In der Apparatur nach Beispiel 1 werden 64,8 g 2,4-Dichloranilin (0,4 Mol), 141,5 g ethanolische 21,2 Gew.%ige Natriumethanolat-Lösung (0,44 Mol) und 500 ml Toluol zum Sieden erhitzt und alles Ethanol abdestilliert. Nach dem Abkühlen auf Raumtemperatur werden 86,5 g Diethyl-malonat (0,4 Mol) zugefügt und nach 1-stündigem Rühren langsam auf 115 °C erhitzt. Innerhalb von 3 h wird bei dieser Reaktionstemperatur der Reaktionsalkohol abdestilliert und nach dem Abkühlen auf 80 °C in Toluol aufgerührt. Nach Neutralisation mit Ameisensäure (0,44 Mol) wird das auskristallisierte Natriumformiat bei 20 °C abfiltriert und das Filtrat über eine Kolonne aufdestilliert.
Nach Abnahme des Toluols und eines geringen Vorlaufes destilliert das Zielprodukt Diethylmalonsäuremonoethylester-(2,4-dichlor)-anilid bei 138 °C/0,5 mbar über.
Ausbeute: 113,9 g (85,7 % d.Th.)
Schmelzpunkt: 26 bis 28 °C
Reinheit: 96 % (G.C.)

| $C_{15}H_{19}Cl_2NO_3$ (332,23) | | | | | |
|---|---|---|---|---|---|
| ber.<br>gef. | C 54,2<br>C 54,2 | H 5,8<br>H 5,8 | Cl 21,4<br>Cl 21,7 | N 4,2<br>N 4,2 | 0 14,5<br>0 14,7 |

Beispiel 17

**Diethylmalonsäure-mono-(2,4-dichlor)-anilid**

25 g (0,075 Mol) des unter Beispiel 16 hergestellten Diethylmalonsäureethylester-mono-(2,4-dichlor)-anilids werden mit 42 g 20 %-iger Kalilauge und 0,5 g des Phasentransfer-Katalysators Aliquat 336 4 bis 5 h zum Sieden erhitzt, bis sich eine einheitliche Phase gebildet hat. Nach Abkühlung auf Raumtemperatur wird mit Ameisensäure neutralisiert und der ausgefallene Niederschlag gut mit Wasser, anschließend mit Heptan verrührt, filtriert und gewaschen.
Auswaage: 18 g (79 % d.Th.)
Schmelzpunkt: 82 bis 86 °C
Reinheit: 96 % (HPLC)

| $C_{12}H_{15}Cl_2NO_3$ (304,17) | | | | | |
|---|---|---|---|---|---|
| ber.<br>gef. | C 51,3<br>C 50,3 | H 5,0<br>H 4,9 | Cl 23,3<br>Cl 22,2 | N 4,6<br>N 4,7 | 0 15,8<br>0 16,1 |

Beispiel 18

**Diethylmalonsäure-bis-(2,4-dichlor)anilid**

In der vorn beschriebenen Glasapparatur werden 129,6 g 2,4-Dichloranilin (0,8 Mol), 283 g ethanolische 21,2 Gew.%-ige Natriumethanolat-Lösung (0,88 Mol) mit 600 ml Toluol zum Sieden erhitzt unter Abdestillation allen Ethanols. Nach Ankühlung auf 70 °C werden 86,5 g Diethyldiethylmalonat (0,4 Mol) zugetropft und 7 h zum Sieden erhitzt, wobei der Reaktionsalkohol abgenommen wird.
Es wird angekühlt, mit 500 ml $H_2O$ versetzt und mit 41 g Ameisensäure (0,88 Mol) unter kräftigem Rühren neutralisiert. Nach Abtrennung der wäßrigen Phase wird die Toluolphase zur Trockne eingeengt. Es wird Ölpumpenvakuum von 0,5 mbar angelegt zur Abdestillation des unverseiften Diethylmalonsäure-ethylester-mono-(2,4-dichlor)anilids und der Destillationsrückstand aus Heptan umkristallisiert.
Schmelzpunkt: 127 bis 128 °C
Reinheit: 98 % (HPLC)

| $C_{19}H_{18}Cl_4N_2O_2$ (448,18) | | | |
|---|---|---|---|
| ber.<br>gef. | C 50,9<br>C 51,3 | H 4,1<br>H 4,2 | N 6,2<br>N 6,1 |

Beispiel 19

**Ethylmalonsäure-mono-(2,4-dichlor)anilid**

In der vorn beschriebenen Glasapparatur werden 64,8 g 2,4-Dichloranilin (0,4 Mol) in 142 g 21,2 Gew.%-iger ethanolischen Natriumethanolat-Lösung (0,44 Mol) gelöst und nach Zugabe von 500 ml Toluol zum Sieden erhitzt, wobei Ethanol abdestilliert wird.
Nach dem Abkühlen auf Raumtemperatur werden 75,3 g Ethylmalonsäure-diethylester (0,4 Mol) zugefügt, die Mischung 1 Std. bei Raumtemperatur und anschließend 1,5 h zum Sieden erhitzt, wobei wiederum der gesamte Reaktionsalkohol azeotrop abdestilliert wird.
Nach dem Abkühlen werden 500 ml Wasser zugesetzt, wieder zum Sieden erhitzt und das Toluol als Azeotrop abdestilliert. Es wird eine Klärfiltration durchgeführt und das Filtrat mit Salzsäure auf pH = 3 angesäuert. Der ausgefallene Niederschlag wird abfiltriert, mit Wasser gewaschen und getrocknet.
Auswaage: 79,6 g (72,1 % d.Th.)
Schmelzpunkt: 141 bis 142 °C
Reinheit: 99,4 % (HPLC)

| $C_{11}H_{11}Cl_2NO_3$ (276,12) | | | | | |
|---|---|---|---|---|---|
| ber.<br>gef. | C 47,9<br>C 47,7 | H 4,0<br>H 4,3 | Cl 25,7<br>Cl 26,5 | N 5,1<br>N 4,8 | 0 17,4<br>0 18,1 |

Beispiel 20

**Ethylmalonsäure-monoethylester-(2,4-dichlor)anilid**

Beispiel 19 wird wie dort angegeben wiederholt, jedoch wird nach Bildung des Anilids wie folgt aufgearbeitet:
Die Toluol-Lösung wird mit konzentrierter Ameisensäure (stöchiom. Menge) neutralisiert, das ausgefallene Natriumformiat abfiltriert und das Filtrat zur Trockne eingeengt:
116,7 g (95,9 % d. Th.).
Dieser Rückstand wird in der Wärme in Cyclohexan weitgehend gelöst, ein unlöslicher Niederschlag (Ethylmalonsäure-bis-(2,4-dichlor)anilid) abfiltriert, und das Cyclohexan-Filtrat soweit eingeengt, daß in der Kälte Kristallisation eintritt. Die Kristalle werden filtriert, mit Heptan gewaschen und getrocknet.
Schmelzpunkt: 54 bis 57 °C
Reinheit: 96 % (G.C.)

| $C_{13}H_{15}Cl_2NO_3$ (304,17) | | | | | |
|---|---|---|---|---|---|
| ber.<br>gef. | C 51,3<br>C 50,4 | H 4,9<br>H 4,8 | Cl 23,3<br>Cl 24,2 | N 4,6<br>N 4,5 | 0 15,7<br>0 15,3 |

Beispiel 21

**Ethylmalonsäure-bis-(2,4-dichlor)anilid**

Beispiel 19 wird wiederholt, jedoch wird die Umsetzung mit 129,6 g 2,4-Dichloranilin (0,8 Mol), 283 g ethanol. 21,2 Gew-%ige Natriumethanolat-Lösung (0,88 Mol) und 75,2 g Ethylmalonsäurediethylester (0,4 Mol) in 500 ml Toluol durchgeführt. Nach Abdestillation des gesamten Ethanols wird die Reaktionsmischung

abgekühlt.
Die Toluolphase wird von dem ausgefallenen Niederschlag dekantiert und der Niederschlag in Wasser, dem die äquivalente Menge Ameisensäure zugesetzt worden ist, aufgerührt. Das so neutralisierte Zielprodukt wird filtriert und je 2 x abwechselnd mit Wasser und Aceton aufgerührt, filtriert, gewaschen und getrocknet.
Schmelzpunkt: 201 bis 203 °C
Reinheit: 96 % (HPLC)

| $C_{17}H_{14}Cl_4N_2O_2$ (420,13) | | | |
|---|---|---|---|
| ber. | C 48,6 | H 3,3 | N 6,7 |
| gef. | C 48,1 | H 3,5 | N 6,3 |

Beispiel 22

**2,4-Dichloranilin-Na-Salz**

In einen 1 l-Mehrhalskolben werden 40,0 g 2,4-Dichloranilin (0,246 Mol), 44,2 g 30 Gew.-%ige Natriummethanolat-Lösung (0,246 Mol) und 300 ml Cyclohexan eingefüllt und zum Sieden erhitzt, wobei das Methanol als Azeotrop abgenommen wird. Nach Abdestillation des gesamten Methanols wird abgekühlt, der auskristallisierte Niederschlag abfiltriert, mit Cyclohexan nachgewaschen und getrocknet.
Ausbeute: 41 g (90,5 % d.Th.)
Schmelzpunkt: > 200 °C
Na-Gehalt [%]: 12,6 % (ber. 12,5 %)

Beispiel 23

**2,4-Dichloranilin-K-Salz**

Entsprechend Beispiel 22, jedoch mit der äquivalenten Menge K-Methanolat-Lösung wird das K-Salz in über 90 % Ausbeute erhalten.
Schmelzpunkt: > 200 °C
K-Gehalt wie berechnet

Beispiel 24

Entsprechend Beispiel 22 werden die Na- bzw. K-Salze von 3-Chloranilin, 3,4-Dichloranilin und 2,6 Dichloranilin hergestellt.
Die Schmelzpunkte liegen über 200 °C, der Na- bzw. K-Gehalt ist wie berechnet.

**Patentansprüche**

1. Verfahren zur Herstellung von Monoester-monoamiden, Monosäure-monoamiden und Bis-amiden der ein- und zweifach substituierten Malonsäuren der allgemeinen Formel

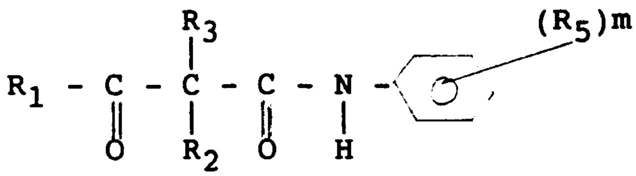

durch Umsetzung von ein- und zweifach substituierten Malonsäuredialkylestern der Formel (II) mit Aminen der Formel (III)

(II) 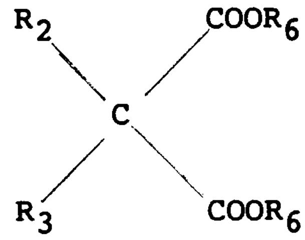 (III) 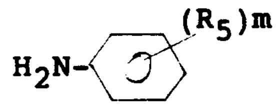

worin

$R_1$ = O-Alkyl ($C_1$-$C_6$), OH, $O^-$ Alkali $^+$,

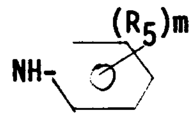

$R_2$ = Alkyl ($C_1$-$C_6$), Alkoxy ($C_1$-$C_6$), Aryl
$R_3$ = H, gleiche oder verschiedene $R_2$
$R_2$ + $R_3$ = $-(R_4CR_4)_n-$, n = 2-5
$R_4$ = H, $CH_3$, Halogen
$R_5$ = Halogen, Alkyl ($C_1$-$C_6$), Alkoxy ($C_1$-$C_6$), $N(R_6)_2$, Cyan, Acyl, Nitro, Thioalkyl ($C_1$-$C_6$)
m = 0 - 5
$R_6$ = Alkyl ($C_1$-$C_6$)

dadurch gekennzeichnet, daß man

a) stöchiometrische Mengen eines Alkalialkoholates, bezogen auf (III) einsetzt,

b) zur Herstellung der Zielprodukte Malonmonoester-monoanilid und Malon-bisanilid das Reaktionsprodukt mit Säuren neutralisiert und dann zu den Zielprodukten aufarbeitet,

c) zur Herstellung des Zielproduktes Malonsäure-monoanilid im Reaktionsgemisch mit Wasser die Estergruppe verseift, die wäßrige Phase ansäuert und das ausgeschiedene Zielprodukt isoliert.

**2.** Verfahren nach Anspruch 1, dadurch gekennzeichnet, daß man Alkalialkoholat in 85 bis 130 %, vorzugsweise 95 bis 110 % der stöchiometrischen Menge, bezogen auf (III), einsetzt.

**3.** Verfahren nach Anspruch 1, dadurch gekennzeichnet, daß man die Alkalialkoholate in fester Form oder als Lösung im betreffenden Alkohol einsetzt.

**4.** Verfahren nach Anspruch 1, dadurch gekennzeichnet, daß man die vorher hergestellten Alkalisalze der Aniline direkt einsetzt.

**5.** Verfahren nach Anspruch 1, dadurch gekennzeichnet, daß man in Lösung inerter aliphatischer oder aromatischer Kohlenwasserstoffe arbeitet.

**6.** Verfahren nach Anspruch 1, dadurch gekennzeichnet, daß man in einem Temperaturbereich bis 150 °C arbeitet.

**7.** Die Stoffe

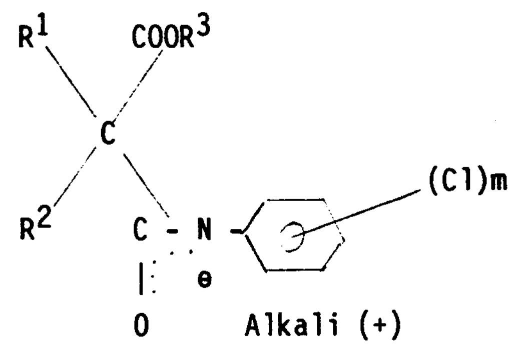

mit

| | |
|---|---|
| $R^1$ | = $CH_3$, $C_2H_5$ |
| $R^2$ | = H oder $R^1$ |
| m | = 1 bis 3 |
| $R^3$ | = Alkali, $CH_3$, $C_2H_5$, |

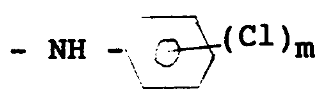

Alkali = Na oder K

**8.** Die Stoffe

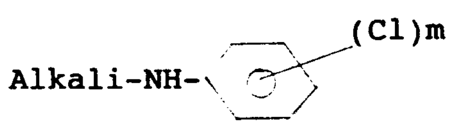

m = 1 bis 5

## Claims

**1.** Method for the preparation of monoester-monoamides, monoacid-monoamides and bis-amides of once and twice substituted malonic acids of the general formula:

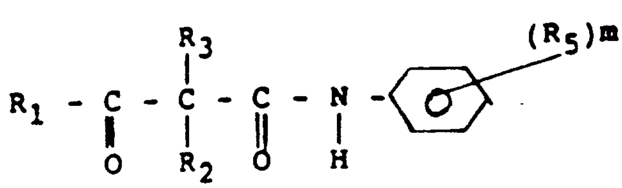

by reaction of once and twice substituted malonic acid dialkylesters of the formula (II) with amines of the formula (III)

(II) 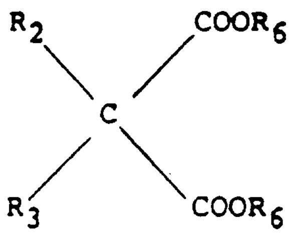

(III) 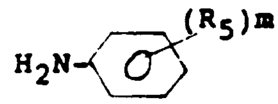

wherein

$R_1$ = O-Alkyl ($C_1$-$C_6$), OH, $O^-$ Alkali $^+$,

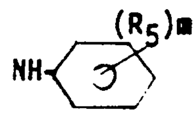

$R_2$ = Alkyl ($C_1$-$C_6$), Alkoxy ($C_1$-$C_6$), Aryl

$R_3$ = H, same or different $R_2$

$R_2$ + $R_3$ = $-(R_4CR_4)_n-$, n = 2-5

$R_4$ = H, $CH_3$, Halogen

$R_5$ = Halogen, Alkyl ($C_1$-$C_6$), Alkoxy ($C_1$-$C_6$), $N(R_6)_2$, Cyano, Acyl, Nitro, Thioalkyl ($C_1$-$C_6$)

m = 0 - 5

$R_6$ = Alkyl ($C_1$-$C_6$)

characterised in that

a) stoichiometric amounts of an alkali alcoholate related to (III) are employed,

b) the reaction product is neutralised with acids, for the production of the target product malonomonoester-monoanilide and malono-bisanilide, and is then worked up to the target product,

c) for the production of the target product malonic acid-monoanilide, there is saponification of the ester groups in the reaction mixture with water, the aqueous phase is acidified and the target products which is separated out is isolated.

**2.** Method according to claim 1, characterised in that alkali alcoholate is employed in 85-130%, preferably 95-110% of the stoichiometric amount related to (III).

**3.** Method according to claim 1, characterised in that the alkali alcoholate is employed in solid form or as solution in the alcohol being formed.

**4.** Method according to claim 1, characterised in that the previously produced alkali salt of the aniline is employed directly.

**5.** Method according to claim 1, characterised in that working takes place in solution of inert aliphatic or aromatic hydrocarbons.

**6.** Method according to claim 1, characterised in that working takes place in a temperature range up to 150 °C.

**7.** The substances

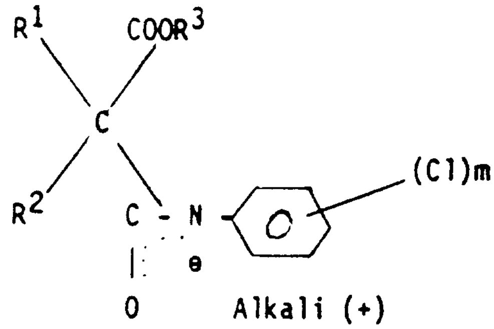

with

| | |
|---|---|
| $R^1$ | = $CH_3$, $C_2H_5$ |
| $R^2$ | = H or $R^1$ |
| m | = 1 to 3 |
| $R^3$ | = Alkali, $CH_3$, $C_2H_5$, |

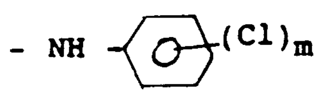

Alkali = Na or K

**8.** The substances

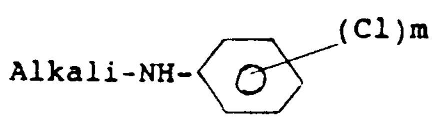

m = 1 to 5

**Revendications**

**1.** Procédé de préparation de monoester-monoamides, de monoacide-monoamides et de bis-amides, d'acides maloniques substitués une ou deux fois, de formule générale :

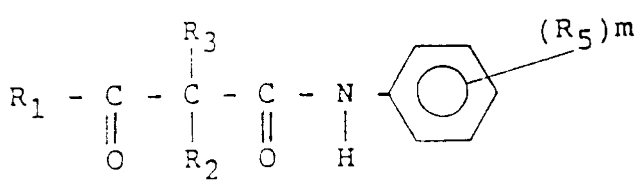

par la réaction d'esters dialkyliques de l'acide malonique, substitués une ou deux fois, de formule (II) avec des amines de formule (III)

(II) 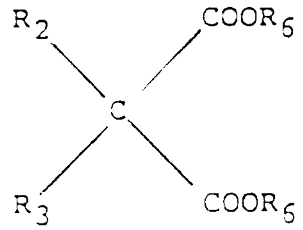

(III) 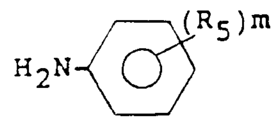

dans lesquelles

$R_1$ représente un groupe O-alkyle en $C_1$ à $C_6$, OH, $O^-$ métal alcalin$^+$,

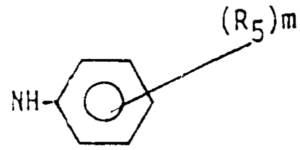

$R_2$ représente un groupe alkyle en $C_1$ à $C_6$, alcoxy en $C_1$ à $C_6$, aryle,
$R_3$ représente H, des groupes $R_2$ identiques ou différents,
$R_2$ + $R_3$ = $-(R_4CR_4)_n-$, n valant 2 à 5
$R_4$ représente H, un groupe $CH_3$, un atome d'halogène
$R_5$ représente un atome d'halogène, un groupe alkyle en $C_1$ à $C_6$, alcoxy en $C_1$ à $C_6$, $N-(R_6)_2$, cyano, acyle, nitro, thioalkyle en $C_1$ à $C_6$,
m vaut 0 à 5
$R_6$ représente un groupe alkyle en $C_1$ à $C_6$

procédé caractérisé en ce que :

a) on utilise des quantités stoéchiométriques d'un alcoolate alcalin, par rapport à (III),
b) pour préparer les produits visés que sont le monoanilide de monoester malonique et le bisanilide malonique, on neutralise le produit de la réaction avec des acides, puis on le transforme en les produits visés ,
c) pour préparer le produit visé qu'est le monoanilide d'acide malonique on saponifie avec de l'eau, dans le mélange réactionnel, le groupe ester, on acidifie la phase aqueuse et l'on isole le produit visé qui s'est séparé.

**2.** Procédé selon la revendication 1, caractérisé en ce qu'on utilise l'alcoolate alcalin en une quantité représentant 85 à 130 %, avantageusement 95 à 110 % de la quantité stoéchiométrique, par rapport à (III).

**3.** Procédé selon la revendication 1, caractérisé en ce qu'on utilise les alcoolates alcalins sous forme solide ou sous forme d'une solution dans l'alcool correspondant.

**4.** Procédé selon la revendication 1, caractérisé en ce qu'on utilise directement les sels alcalins préalablement préparés, des anilines.

**5.** Procédé selon la revendication 1, caractérisé en ce qu'on travaille dans une solution dans des hydrocarbures aliphatiques ou aromatiques inertes.

**6.** Procédé selon la revendication 1, caractérisé en ce qu'on travaille dans un intervalle de la température allant jusqu'à 150 °C.

**7.** Les matières :

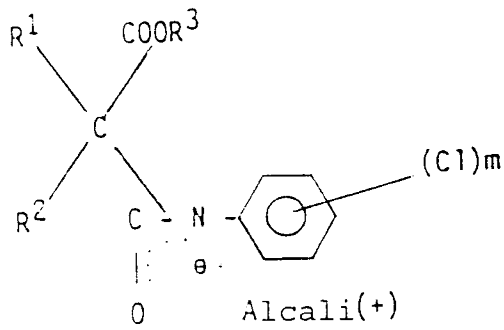

formule dans laquelle

$R^1$ représente un reste $CH_3$ ou $C_2H_5$

$R^2$ représente H ou un reste $R^1$;

m vaut 1 à 3

$R^3$ représente un métal alcalin, $CH_3$, $C_2H_5$,

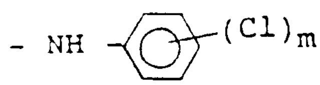

alcali représente Na ou K.

**8.** Les matières :

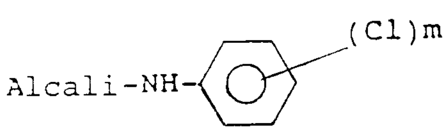

dans lesquelles m vaut 1 à 5.